# EUROPEAN PATENT SPECIFICATION

(11) **EP 4 157 066 B1**
(45) Date of publication and mention of the grant of the patent: **06.08.2025**
(21) Application number: 21722905.3
(22) Date of filing: 05.05.2021
(51) Int. Cl.: A61B 5/00, A61N 1/372, A61B 5/0205, A61B 5/0245, A61B 5/11, A61N 1/39

(54) **SYSTEM FOR GENERATING AN ALERT FOR A SYSTEMIC INFECTION**
SYSTEM ZUR ERZEUGUNG EINER WARNUNG FÜR EINE SYSTEMISCHE INFEKTION
SYSTÈME POUR GÉNÉRER UNE ALERTE EN CAS D'INFECTION SYSTÉMIQUE

(30) Priority: 26.05.2020 US 202063029718 P; 15.09.2020 EP 20196091
(43) Date of publication of application: 05.04.2023
(73) Proprietor: BIOTRONIK SE & Co. KG, 12359 Berlin (DE)
(72) Inventor: DOERR, Thomas, 12437 Berlin (DE); MUESSIG, Dirk, West Linn, OR 97068 (US); REDDY, Ravi, Portland, OR 97221 (US); WHITTINGTON, R. Hollis, Portland, OR 97202 (US); DIEM, Bjoern Henrik, 14197 Berlin (DE)
(74) Representative: Biotronik Corporate Services SE
(86) International application number: PCT/EP2021/061769
(87) International publication number: WO 2021/239410

(56) References cited:
- WO-A1-2009/055865
- WO-A2-2009/138976
- US-A1- 2008 064 980
- US-A1- 2008 262 323

## Description

The present invention concerns a system for generating an alert for a systemic infection of a patient.

A system of this kind comprises an implantable medical device configured to measure at least one physiological parameter, a remote monitoring system configured to receive information from the implantable medical device, and an information system configured to communicate with said remote monitoring system.

An implantable medical device of the type described herein may for example be a sensing system that can be implanted in a patient's vessel, for example to measure a parameter such as a body temperature or the heart rate of the patient. The sensing system can be used to monitor a patient's condition, for example for observing and diagnosing a course of disease, wherein the implantable medical device is designed to communicate with the remote monitoring system (located outside the patient) to transmit the measurement results to the remote monitoring system after measurement by the implantable medical device. Alternatively, the implantable medical device may be designed as an implantable stimulation device, for example with a pacemaker or neuro-stimulation function, or alternatively a diagnostic device, such as a diagnostic patch, or a wearable device.

Generally, the implantable medical device may be any active medical device which is to be implanted into a patient and, in an implanted state, performs a function, for example a pacing function or a monitoring function, within the patient, wherein the implantable medical device generally shall remain within the patient over a prolonged period of time, for example several months or even years. The implantable medical device generally comprises a functional device in the form of an electronics unit, which is formed for example by a processor and serves to perform a function in an active operational state of the medical device, for example a measurement function to measure a physiological parameter of a patient, for example the heart rate. To operate the functional device, the implantable medical device generally comprises an energy storage element, particularly in the form of an electric battery, which feeds the functional device and supplies it with power in its operational state.

Implantable medical devices may be operated in cooperation with a remote monitoring system, comprising for example one or multiple monitoring devices which may serve to monitor operation of the implantable medical device at the home of a patient and hence remotely from a healthcare environment such as a hospital. Such remote monitoring system generally is in communication with an information system (also denoted as patient information system), such as a centralized service center, which may for example process data received from the implantable medical device via the remote monitoring system and may provide information derived from the data to healthcare personnel, such as a physician in a hospital or the like. The information system hence in cooperation with the remote monitoring system allows for a continuous monitoring of the state of the patient, for example a cardiac function of the patient if the implantable medical device is a cardiac device, such as a cardiac pacemaker or a defibrillator.

If the implantable medical device is a cardiac device, it generally provides information relating to a cardiac function and hence allows for a monitoring of a cardiac state of health. There however may be a desire to derive further information to allow for an enhanced monitoring using an existing setup, in particular an existing implantable medical device in cooperation with a remote monitoring system and an information system

WO2009/138976 discloses a system for generating an alert for a systemic infection of a patient.

It is an object of the instant invention to provide a system which allows for an enhanced monitoring of a patient, in particular relating to a general state of the patient.

This object is achieved by means of a system comprising the features of claim 1 defining the present invention.

Accordingly, at least one of the implantable medical device, the remote monitoring system and the information system is configured to analyze information relating to said at least one physiological parameter to determine an alert signal for a systemic infection of the patient based on a state of the at least one physiological parameter, wherein at least one of the implantable medical device, the remote monitoring system and the information system is further configured to generate an alert message to be provided to a specified destination based on said alert signal.

In the face of the ongoing COVID-19 pandemic, early detection of infected patients and early quarantine is essential to contain the pandemic. There hence is a desire to provide means to allow for a generation of an alert in case a substantial risk for a systemic infection exists, making use of devices and systems that already are installed and are functional to allow for a monitoring of patients, for example cardiac patients belonging to a particular risk group for systemic infections such as COVID-19.

Although described in the context of COVID-19, the instant solution may be applicable also to other systemic infections in particular in high risk populations, wherein patients with implants often belong to a particular risk group.

According to the solution described herein, a system comprises an implantable medical device in the shape of an active electronic implant and a remote monitoring system, wherein the implantable medical device is communicatively connected to the remote monitoring system in an implanted state. The implantable medical device is implanted e.g. in a human or an animal body, wherein the implantable medical device is equipped with at least one or more sensors to detect one or more physiologic parameters, which typically deviate from a normal value in case of a systemic infection of the patient. The implantable medical device is configured to transmit one or more of the physiologic parameters and/or information derived from the physiologic parameters to the remote monitoring system for example in a pre-specified time period.

Within the system, one of the implantable medical device, the remote monitoring system and the information system is configured to analyze information relating to the at least one physiological parameter to determine an alert signal for a systemic infection of the patient based on the at least one physiological parameter. Hence, the implantable medical device, the remote monitoring system or the information system is enabled to analyze the at least one physiological parameter in order to derive an alert in case it is found that the at least one physiological parameter indicates an increased likelihood for a systemic infection, for example based on a change of the at least one physiological parameter.

If it is found that an increased likelihood for a systemic infection exists within a patient, the alert signal is generated, and based on the alert signal an alert message is transmitted to a specified destination, for example to the patient or to a person familiar with the patient, for example a relative or local healthcare personnel, for example a physician involved in the treatment of the patient. By means of the alert message hence for example a relevant party, for example a physician, is informed of a risk of a systemic infection of the patient, such that the third party may attend to the patient and may confirm or disconfirm the alert. If in fact a systemic infection of the patient is found, medical treatment may be started, possibly in connection with a quarantine of the patient.

The system hence allows, based on an implanted medical device serving for example a cardiac function, to monitor a patient for a potential risk of a systemic infection, hence allowing for fast countermeasures to treat the patient and to avoid a potential spread of the systemic infection, for example COVID-19.

The system may for example comprise a module for the automatic generation of a (first level) alert signal, wherein the alert signal is based on the transmitted data from the implant to the remote monitoring system. The module may for example be part of the remote monitoring system or the information system, which hence are enabled to process data received from the implantable medical device in order to generate the alert signal. The alert signal may for example be set to a first value in case the patient is potentially affected by the systemic infection, and the alert signal may be set to a second value if the patient is likely not affected by the systemic infection. The module for the automatic generation of the alert signal may for example be directly or indirectly connected to the information system. The information system may be configured for example to inform the patient or a person familiar with the patient in case the alert signal is set to the first value in order to prompt the patient or the person familiar with the patient to confirm the status of the patient and to initiate a medical action.

In one embodiment, the implantable medical device may be configured to transmit information relating to said at least one physiological parameter to the remote monitoring system once a day, twice a day, three times a day, four times a day or within other regular periods throughout a day. If a pre-specified time period for the transmission is e.g. one day, it may be preferable that a deviation from this time period is within a range of +/- 12 hours.

In one embodiment, the implantable medical device comprises a communication device for communicating with the remote monitoring system using a predefined communication protocol for transmitting information relating to said at least one physiological parameter to the remote monitoring system. The predefined communication protocol may for example be MICS (Medical Implant Communication Service), BLE (Bluetooth Low Energy), Zigbee, or a communication protocol of a band telemetry. The communication protocol may offer for a low power, low data rate, close proximity wireless data transmission.

In one embodiment, the implantable medical device is an implantable pulse generator (IPG) such as an implantable cardiac pacemaker, an implantable cardioverter-defibrillator (ICD), a cardiac resynchronization therapy device (CRT) like a cardiac resynchronization therapy pacemaker or a cardiac resynchronization therapy defibrillator, a neuro stimulator or an implantable loop recorder. The implantable medical device constitutes an active electronic implant which is configured to communicate, in an implanted state within a patient, with the remote monitoring system external to the patient, for example using a short range communication technology.

In one embodiment, the at least one physiologic parameter is one or more of the following parameters: the body temperature, the heart rate, the mean heart rate, the heart rate at rest, the respiration rate for normal breathing patterns, the respiration rate for abnormal breathing patterns, the presence of breathing pauses or apnea events (cessation of respiration), the relationship between respiration effort and respiration activity (respiration depth), other respiration parameters, a patient activity parameter and any combination of these parameters.

The body temperature may for example be measured by means of a temperature sensor of the implantable medical device.

The heart rate of the patient may be measured according to for example an electrocardiogram signal as sensed by the implantable medical device. Herein, the physiological parameter may be the actual heart rate. Alternatively or in addition, the physiological parameter may be a mean heart rate value associated with an average of the heart rate over a predefined period of time, for example multiple hours. Yet alternatively or in addition, the physiological parameter may be the heart rate at rest, corresponding to the measured heart rate in a rest period of the patient, determined for example by means of a sensed activity of the patient, for example using a motion sensor in the shape of an accelerometer or the like. The mean heart rate at rest may be averaged for example over a rest period.

Respiration parameters or a body activity parameter may for example be derived by motion signals, for example sensed by a motion sensor such as an accelerometer.

Other physiological parameters may be taken into account, wherein generally all physiological parameters measured or derived from measurements of the implantable medical device may be used alone or in combination for determining said alert signal.

In one embodiment, the implantable medical device is configured to generate said alert signal based on said at least one physiologic parameter and to transmit information relating to said alert signal to the remote monitoring system. Hence, it is the implantable medical device itself which generates an alert in case a risk for a systemic infection of the patient is found. The alert signal may be transmitted to the remote monitoring system, such that the remote monitoring system may further process the alert signal.

In another embodiment, the remote monitoring system is configured to generate said alert signal based on information relating to said at least one physiologic parameter as received from the implantable medical device and to transmit information relating to said alert signal to the information system. In this case, hence, the remote monitoring system comprises a module for the automatic generation of the alert signal, wherein the alert signal may be forwarded to the information system for further processing.

In yet another embodiment, the information system is configured to generate said alert signal based on information relating to said at least one physiological parameter as received from the remote monitoring system. In this case, hence, the information system comprises a module for the automatic generation of the alert signal.

Based on the alert signal, the alert message is generated and transmitted to a specified destination, for example a specified communication terminal associated with the patient or a person familiar with the patient. The alert message herein may be generated by the same entity that generates the alert signal. Alternatively, the alert message may be generated by a different entity than the one that generates the alert signal. For example, if the alert signal is generated by the implantable medical device itself, the alert message may be generated and transmitted by the remote monitoring system or the information system. If the alert signal is generated by the remote monitoring system, the alert message may be generated by the remote monitoring system or by the information system for transmission to the specified destination.

In one embodiment, the information system is an automated information system delivering patient information to the patient.

In one embodiment, the delivery of the patient information by the information system is based on one or more of the following services: a phone call, a video message, an e-mail message, a short message (SMS) or messages from other messenger services.

In one embodiment, the information system is supported by a "staffed service". Hence, data processing may take place substantially automatically at to the information system, wherein staff of the information system, for example healthcare personnel, may be involved in the data processing and in particular in the delivery of patient information to the patient, for example for initiating a phone call to the patient.

In one embodiment, the remote monitoring system comprises a subsystem for the documentation of the results of an alert verification. The alert verification may be an automated process or a manual process using medically trained personnel. Documented results of the alert verification may be used to automatically evaluate and/or improve the performance of the module for the automatic generation of the alert signal, e.g. by machine learning.

In one embodiment, the information system is configured to transmit said alert message to said destination using a dedicated communication path. The dedicated communication path herein may be different than a default communication path which is used by the information system to provide information not relating to the alert message for example to the patient or to medical personnel. For example, in case of a positive alert a patient or a person familiar with the patient, for example a physician, may be informed about the positive alert by means of the alert message, wherein the alert message may be sent to the patient or the person familiar with the patient via a communication path which specifically is dedicated to the transmission of an infection alert. For example, the alert message may be sent to a dedicated communication terminal, for example to a smart phone of the patient or the person familiar with the patient, for example via a smart phone application (in short app) specifically designed to receive and process alert messages relating to infection alerts. Information not relating to an infection alert in contrast may be provided via another default communication path, for example via a web interface which the patient or a physician may access to obtain the information at the information system.

In one embodiment, the implantable medical device, the remote monitoring system or the information system is configured, for analyzing the at least one physiologic parameter, to compare the at least one physiological parameter or a parameter value derived from the at least one physiological parameter to a threshold and to generate the alert signal based on the comparison.

Generally, in case of a systemic infection an increase in the body temperature will be correlated with an increase in the heart rate. Hence, based on the heart rate conclusions can be drawn with respect to a change in the body temperature, wherein an alert for a possible systemic infection may be derived if the heart rate, in particular the heart rate at rest, increases and exceeds a certain threshold. For example, in case of a systemic infection, for example COVID-19, it may be found that an increase of the body temperature by 1°C causes an increase of the heart rate by about 10 bpm. A heart rate increase, in particular an increase of the rested heart rate, hence may be indicative of a potential systemic infection and can be used to trigger an infection alert signal.

In one embodiment, the mean value of the heart rate, for example averaged over a predefined time period, for example multiple hours, is compared to the threshold. Alternatively, the heart rate at rest, as measured during a rest period of the patient and potentially averaged over at least a portion of the rest period, may be compared to the threshold in order to generate the alert signal.

The threshold herein may be fixed or may depend on previous values of the heart rate or the heart rate at rest.

If the threshold is fixed, it may for example have a value in a range between 90 bpm and 120 bpm, for example 90 bpm, 95 bpm, 100 bpm or 105 bpm. The threshold in this case may be programmed by programming the implantable medical device, the remote monitoring system or the information system.

If the threshold is adaptive in that it depends on previous values of the heart rate or the heart rate at rest, the threshold may for example lie in a range between 110% and 150% of an average of the heart rate or the heart rate at rest as measured over a predefined, previous time period. The time period may for example have a length of multiple days, for example 5 days, 10 days, 15 days, 20 days or 30 days. The threshold is then set based on the average heart rate or the heart rate at rest, for example to a value corresponding to 110%, 120% or 130% of the average heart rate in the previous time period.

By evaluating the heart rate or the heart rate at rest, an alert for a systemic infection may be generated even in case the implantable medical device does not comprise a temperature sensor. This makes use of the finding that the body temperature and the heart rate, in particular the heart rate at rest, may be correlated, an increase in the body temperature due to a systemic infection generally corresponding to an increase of the heart rate, in particular the heart rate at rest.

In addition or alternatively to comparing the heart rate or the heart rate at rest to a fixed or adaptive threshold, a slope value may be derived from the heart rate or the heart rate at rest, the slope value being indicative of a slope in the change of the heart rate or the heart rate at rest, wherein the slope may be analyzed to generate the alert signal.

In one embodiment, the longevity of the active electronic implant using daily data transmissions to the remote monitoring system, is reduced by (at most) 10%, preferably by 5% and more preferably by 1% compared to an implant using quarterly data transmission to the remote monitoring system.

In another aspect, a method for generating an alert for a systemic infection of a patient is provided, the method comprising: measuring, using an implantable medical device, at least one physiological parameter; receiving, by a remote monitoring system, information from the implantable medical device; communicating, by an information system, with said remote monitoring system; analyzing, by at least one of the implantable medical device, the remote monitoring system and the information system, information relating to said at least one physiological parameter to generate an alert signal for a systemic infection of the patient based on a state of the at least one physiological parameter; and generating, by at least one of the implantable medical device, the remote monitoring system and the information system, an alert message to be provided to a specified destination based on said alert signal.

The advantages and advantageous embodiments described above for the system equally apply also to the method, such that it shall be referred to the above.

A general non-claimed method for an early infection detection using a system of the type described above may comprise the following steps:
- recording one or more physiologic parameters associated with a systemic infection of the patient using the implantable medical device;
- transmitting the physiologic parameter(s) or an information derived from the physiologic parameter(s) at least once a day from the implantable medical device to the remote monitoring system;
- automatically generating a first level alert in case of suspicious infection;
- transferring the first level alert via a patient information system to the patient or a person familiar with the patient;
- verifying the infection symptoms of the patient; and
- taking medical action in case of positive symptoms of the patient.

The idea behind the invention shall subsequently be explained in more detail by referring to the embodiments shown in the figures. Herein:
- Fig. 1: shows a schematic view of a system for generating an alert of a systemic infection of a patient;
- Fig. 2: shows a schematic view of an implantable medical device;
- Fig. 3: shows a flowchart for generating an alert signal and for transmitting an alert message based on the alert signal;
- Fig. 4A: shows a graph of the mean daily temperature of a patient over a period of time;
- Fig. 4B: shows a graph of an activity parameter of the patient during the period of time;
- Fig. 4C: shows a graph of the daily mean heart rate over the period of time;
- Fig. 4D: shows a graph of the daily resting heart rate over the period of time; and
- Fig. 5: shows a graphical visualization of a percentage of alert messages depending on a threshold for generating an alert signal.

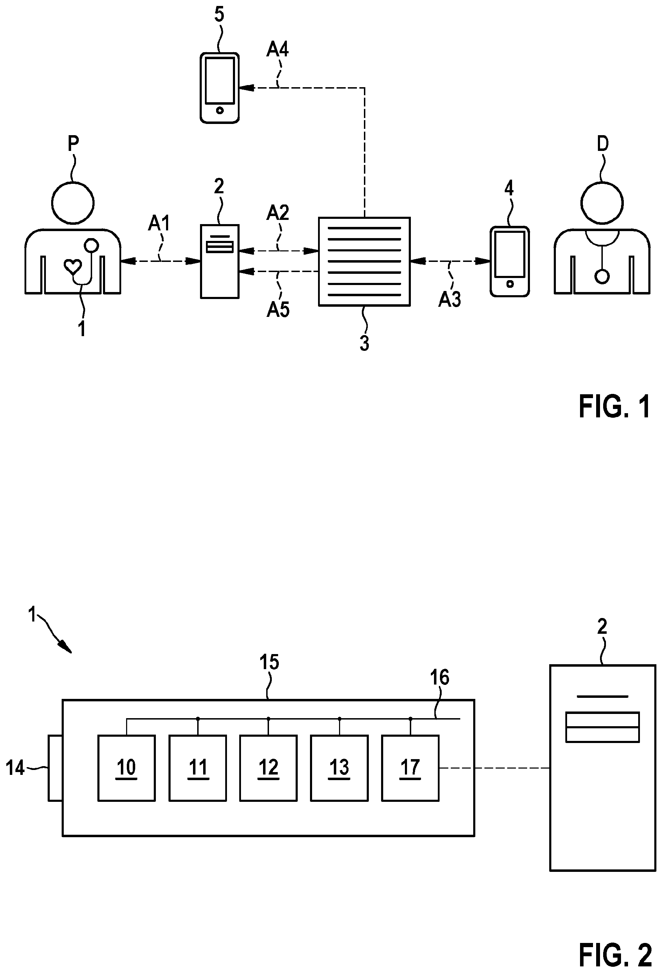

Fig. 1 shows a schematic illustration of a system for generating an alert for a systemic infection of a patient P.

The system comprises an implantable medical device 1 implanted in a patient P, the implantable medical device 1 for example being a cardiac stimulation devices such as a pacemaker device or a defibrillator device, for example an implantable pulse generator (IPG) such as an implantable cardiac pacemaker, an implantable cardioverter-defibrillator (ICD), a cardiac resynchronization therapy device (CRT) such as a cardiac resynchronization therapy pacemaker or a cardiac resynchronization therapy defibrillator, a neuro stimulator or an implantable loop recorder.

The implantable medical device 1 constitutes an active electronic implant which is configured to communicate, in an implanted state within a patient P, with a remote monitoring system 2 external to the patient P. The remote monitoring system 2 may comprise one or multiple portable devices, such as mobile telecommunication devices, which are placed in the home of the patient P and serve to communicate with the implantable medical device 1 to exchange information with the implantable medical device 1 using a communication path A1, for example according to a communication technology using the MICS protocol, the BLE protocol or the Zigbee protocol.

The remote monitoring system 2 is in communication connection with an information system 3 by means of a communication path A2, the communication path A2 being established for example via a public communication network such as the Internet or a telecommunications network, for example a mobile communication networks such as a 2G, 3G, 4G or 5G telecommunications network. The remote monitoring system 2 generally functions as a relay to transmit information from the implantable medical device 1 to the information system 3, or from the information system 3 to the implantable medical device 1.

The information system 3 may be configured to process data received from the implantable medical device 1 via the remote monitoring system 2. Processed data may be provided to an access device 4 of healthcare personnel, for example a physician D, via a communication path A3, for example by means of a web interface to which the access device 4 may connect for accessing the data on the information system 3.

The implantable medical device 1 generally is configured to perform a function in a patient over a prolonged period of time, such as a measurement function or a cardiac or neuronal stimulation function for the purpose of therapy. For example, the medical device 1 shall remain in a patient for multiple years in order to record measurement data during the lifetime of the medical device 1 and to communicate with the remote monitoring system 2, so that the measurement data may be used to diagnose or monitor the condition of the patient.

Such an implantable medical device 1 generally is small in size. As schematically shown in Fig. 2, the medical device 1, for example, comprises a housing 15 encompassing an electronic functional device 10 which is formed, for example, by a processor and serves to perform a predetermined function, for example a measuring function or a therapy function. The medical device 1 in addition comprises a memory 11 e.g. in the form of a RAM (Random Access Memory), (optionally) a wake-up device 12, an energy storage 13, for example in the form of a battery, and a communication device 17 for communicating with remote monitoring system 2. The different functional units are encapsulated in the housing 15 in a fluid-tight manner and are interconnected for example by a bus system 16 for a data exchange in between the different devices.

The medical device 1 in addition, in the shown embodiment, comprises a measurement sensor 14, which is used together with the functional device 10 to perform a measurement in order to record one or multiple physiological parameters, for example to measure the patient's body temperature, the heart rate, the heart rate at rest, respiration parameters, and/or an activity parameter. Measurements may be repeatedly performed in predetermined measurement periods, with measurement data being stored e.g. temporarily in the memory 11 during a measurement and communicated to the remote monitoring system 2 via the communication device 17.

The measurement sensor 14 may for example be an electrode which is in contact with tissue in the vicinity of the implanted medical device 1, or which is placed on a lead extending from the medical device 1 towards a location of interest. By means of the measurement sensor 14 for example an electrocardiogram signal may be recorded, the electrocardiogram signal allowing for a sensing of the heart rate and the heart rate at rest.

Because the medical device 1 has small dimensions, the size of the energy storage 13 is also necessarily limited. Because the medical device 1 is to remain in a patient and be operative for a prolonged period of time, for example several years, it is desired that the medical device 1 operates energy-efficiently, thus requiring little power, but still functions reliably to perform one or more predetermined functions. In order to reduce the energy consumption of the medical device 1, in one embodiment the functional device 10 possibly does not operate continuously and at all times, but may be switched from a switched-off state to an operational state when required in order to carry out a function in the operational state. In the switched-off state the functional device 10 is shut down and causes no or only a very limited power consumption, so that in the switched-off state of the functional device 10 the system of the medical device 1 exhibits a low overall power consumption. In order to transfer the functional device 10 from the switched-off state to the operational state, the wake-up device 12 is provided, which serves to switch on the functional device 10 based on a signal provided e.g. from an external activator device, such as the remote monitoring system 2.

Using the implantable medical device 1 in cooperation with the remote monitoring system 2 and the information system 3, an alert signal for a systemic infection of a patient P may be generated. In particular, one or multiple physiological parameters as measured by the implantable medical device 1 may be processed in order to derive the alert signal, wherein based on the alert signal an alert message may be generated and forwarded to the patient P or a person familiar with the patient P, such as a relative of the patient P or a physician D.

In particular, based on a state of one or multiple physiological parameters, such as the body temperature or the heart rate, conclusions may be drawn with respect to a risk of a systemic infection of the patient P. If it is found that a risk for a systemic infection of the patient P exists, the alert signal may be generated, wherein based on the alert signal an alert message is transmitted to a specified destination, for example to the patient P or a person familiar with the patient P.

Referring now again to Fig. 1, the alert signal may be generated by the implantable medical device 1, by the remote monitoring system 2 or the information system 3. The alert message, in turn, may be generated by the remote monitoring system 2 or the information system 3, wherein the entity generating the alert signal not necessarily is the same as the entity generating the alert message.

In one embodiment, the implantable medical device 1 is configured to process one or multiple physiological parameters in order to generate the alert signal based on a state of one or multiple of the physiological parameters. The alert signal is then provided to the remote monitoring system 2, which possibly forwards the alert signal to the information system 3. Alternatively or in addition the remote monitoring system 2 or the information system 3 may process information relating to one or multiple of the physiological parameters to generate the alert signal.

The remote monitoring system 2 or the information system 3 may then generate an alert message, and may forward the alert message to the patient P or a person familiar with the patient P.

For example, as illustrated in Fig. 1, the information system 3 may generate an alert message and may forward the alert message via a communication path A4 to a communication terminal 5, for example a smart phone, of the patient P or a person familiar with the patient P. For example, the alert message may be sent to the communication terminal 5 by means of a dedicated application (app) of the communication terminal 5, for example a so-called patient app by means of which the patient P may obtain information from the information system 3.

In addition or alternatively, the information system 3 may send the alert message to the remote monitoring system 2 via a communication path A5.

The transmission of the alert message may generally involve any communication route, such as email, SMS, a messenger app or an automated or staff-assisted phone call.

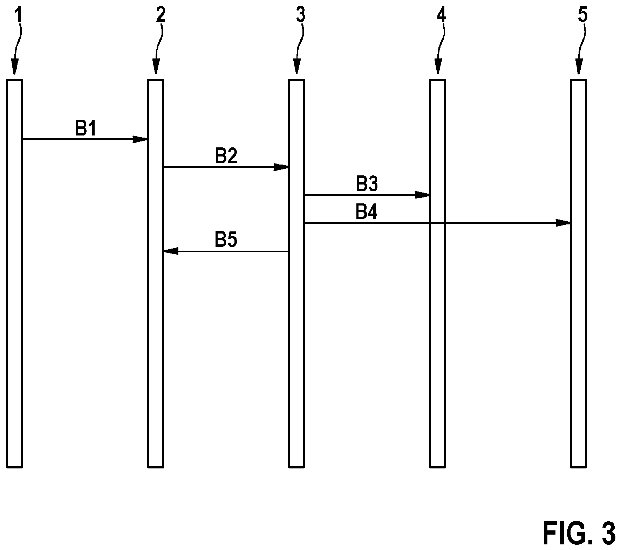

Referring now to Fig. 3 showing a flowchart of a potential message flow, information relating to a measured physiological parameter may be transmitted from the implantable medical device 1 to the remote monitoring system 2 in a data message B1, for example using the MICS, BLE or Zigbee protocol. The remote monitoring system 2 may process the received data and may send a data message B2 to the information system 3, which may further process the data, for example to generate the alert signal and to generate a corresponding alert message.

Information not related to an alert may be provided to an access device 4 for example associated with a physician D, for example by means of a web interface via a data connection B3. In turn, information related to a systemic infection alert may be sent in an alert message B4 to the communication terminal 5, and in an alert message B5 to the remote monitoring system 2, the communication routes for the alert messages B4, B5 hence being different than the data connection B3 for providing general, non-infection related information.

If an alert message is received, for example by a physician familiar with the patient P, the physician may medically attend to the patient P and may confirm or disconfirm a systemic infection. If the systemic infection alert is confirmed, this may be entered into the system, for example at the remote monitoring system 2 or the communication 5, and may be processed by the information system 3, for example, to transmit an automatic infection notification message to an official site, for example an official healthcare office. Treatment of the patient P, for example including a quarantine, may be initiated.

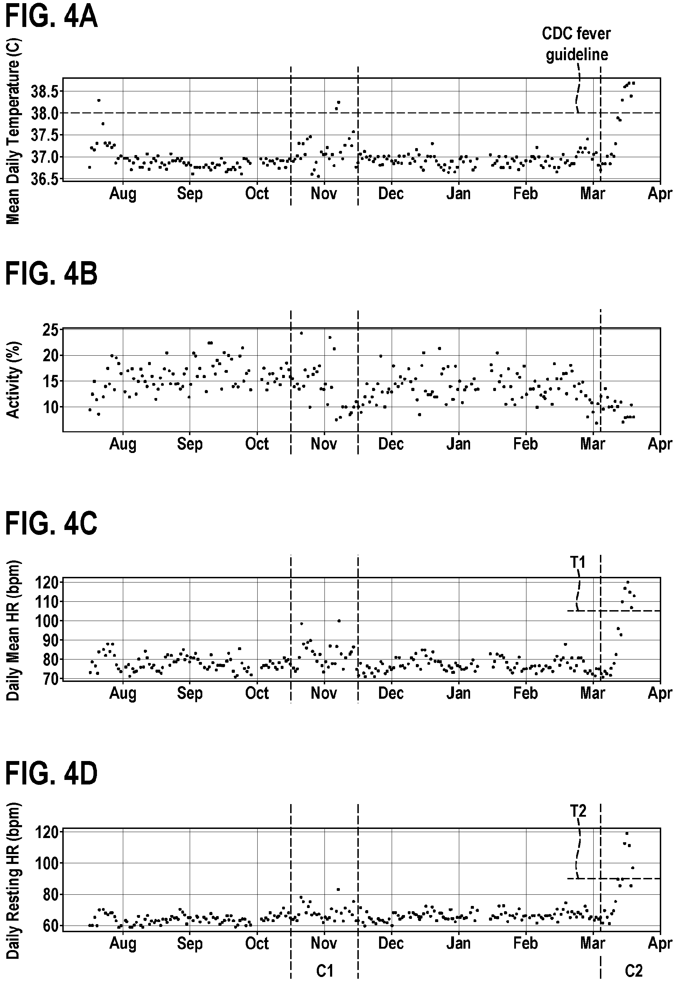

Referring now to Figs. 4A to 4D, one or multiple physiological parameters as sensed by the implantable medical device 1 may be processed in order to derive an alert signal indicative of a possible systemic infection of a patient P, for example a possible COVID-19 infection. Generally, a systemic infection may cause a change in one or multiple physiological parameters, such as the body temperature and the patient's heart rate, such that based on a change in the physiological parameters conclusions may be drawn with respect to a potential systemic infection of the patient P.

Fig. 4A shows the mean daily temperature of a patient P over a prolonged period of time (in the instant case multiple months). Fig. 4B shows a corresponding mean activity of the patient P per day, for example derived from a motion sensor included in the implantable medical device 1, such as an accelerometer or the like. Fig. 4C shows the daily mean heart rate over the period of time, and Fig. 4D shows the daily heart rate at rest over the period of time. Each physiological parameter is represented by one value per day, each physiological parameter hence representing an average value of the respective physiological parameter is averaged over 1 date.

Generally, in case of a systemic infection the body temperature will rise, and in addition also the heart rate will increase. It generally has been found that an increase of 1°C in the body temperature corresponds to an increase of roughly 10 bpm in the heart rate, in case the patient undergoes a systemic infection, in contrast to for example a local infection which may cause an increase in the body temperature, but no or only a limited increase in the patient's heart rate.

This is visible also in Figs. 4A to 4D. Within the series of data points, in a time range C1 it can be observed that increased values for the body temperature can be found (Fig. 4A), corresponding to an increased activity of the patient (Fig. 4B) and a slight increase in the heart rate (Fig. 4C, 4D). In a time range C2 at the end of the data series, in turn, the body temperature is seen to substantially increase (Fig. 4A), at a low activity of the patient P (Fig. 4B), but at a substantially increased heart rate (Fig. 4C) and heart rate at rest (Fig. 4D).

As the heart rate generally increases with the body temperature in case of a systemic infection, the heart rate or the heart rate at rest may be analyzed, possibly in connection with an activity parameter, in order to derive a risk alert for a systemic infection, even without measuring the body temperature, for example in case the implantable medical device 1 does not comprise a temperature sensor.

In particular, as illustrated in Figs. 4C and 4D, if the heart rate (Fig. 4C) or the heart rate at rest (Fig. 4D) exceeds a threshold T1 respectively T2, an alert signal may be generated indicating a positive systemic infection alert. Based on the alert signal, then, an alert message may be generated and forwarded to the patient P or a person familiar with the patient P, as described above according to Figs. 1 to 3.

The threshold T1 for the mean heart rate may be different than the threshold T2 for the heart rate at rest. The threshold T1, T2 in each case may be set as a fixed value, for example within a range between 90 bpm and 120 bpm. Alternatively, the threshold T1, T2 in each case may be set as a percentage of a number of previous values, for example to a value corresponding to a certain percentage, for example within a range of 110% to 150%, of an average of multiple, e.g. consecutive previous values.

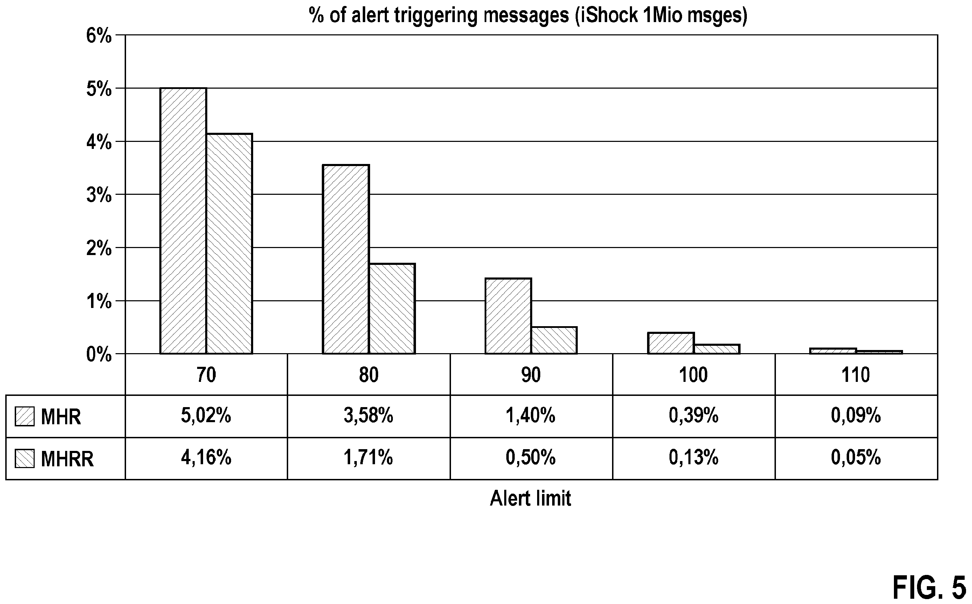

Fig. 5 shows an estimated percentage of alert messages in overall messages sent within a system, dependent on the threshold T1, T2 for the heart rate (MHR) respectively the heart rate at rest (MHRR). If the threshold T1, T2 is set to 70 bpm, a rather large percentage of systemic alert messages may have to be expected within the overall messages sent within the system, possibly increasing the likelihood for a false alarm. If the threshold T1, T2 is chosen larger, the likelihood for an alert message is substantially reduced, as visible from Fig. 5.

The idea underlying the invention is not limited to the embodiments described above, but may be implemented in an entirely different manner.

A system as described herein allows the early medical / pandemic intervention in case of a systemic infection in a high risk patient group.

The system will allow a very short term solution to protect the patients and persons in close contact with the patients during the COVID-19 pandemic (early medical intervention / early quarantine).

### List of reference numerals

- 1: Implantable medical device
- 10: Functional device
- 11: Memory device
- 12: Wake-up device
- 13: Energy storage
- 14: Sensor device
- 15: Housing
- 16: Bus system
- 17: Communication device
- 2: Patient monitoring device
- 3: Monitoring service center
- 4: Access device
- 5: Communication terminal
- A1-A5: Communication path
- B1-B5: Data messages
- C1, C2: time range
- D: Physician
- P: Patient
- T1, T2: Threshold

## Claims

1. A system for generating an alert for a systemic infection of a patient (P), the system comprising: an implantable medical device (1) configured to measure at least one physiological parameter, a remote monitoring system (2) configured to receive information from the implantable medical device (1), and an information system (3) configured to communicate with said remote monitoring system (2), wherein at least one of the implantable medical device (1), the remote monitoring system (2) and the information system (3) is configured to analyze information relating to said at least one physiological parameter to generate an alert signal for a systemic infection of the patient (P) based on a state of the at least one physiological parameter, wherein at least one of the implantable medical device (1), the remote monitoring system (2) and the information system (3) is further configured to generate an alert message (B4, B5) to be provided to a specified destination based on said alert signal, **characterized in that** the information system (3) is configured to transmit said alert message to said destination using a dedicated communication path (A4, A5), wherein the information system (3) is configured to provide information not related to said alert message on a default communication path (A3) different than said dedicated communication path (A4, A5).

2. The system of claim 1, **characterized in that** the implantable medical device (1) comprises a communication device (17) for communicating with the remote monitoring system (2) using a pre-defined communication protocol for transmitting information relating to said at least one physiological parameter to the remote monitoring system (2).

3. The system of claim 2, **characterized in that** the communication protocol is MICS, BLE or Zigbee.

4. The system of one of claims 1 to 3, **characterized in that** said at least one physiological parameter includes at least one of the body temperature, the heart rate, the heart rate at rest, the respiration rate, a body activity parameter.

5. The system of one of the preceding claims, **characterized in that** the implantable medical device (1) is configured to generate said alert signal based on said at least one physiological parameter and to transmit information relating to said alert signal to the remote monitoring system (2).

6. The system of one of the preceding claims, **characterized in that** the remote monitoring system (2) is configured to generate said alert signal based on information relating to said at least one physiological parameter and to transmit information relating to said alert signal to the information system (3).

7. The system of one of the preceding claims, **characterized in that** the information system (3) is configured to generate said alert signal based on information relating to said at least one physiological parameter.

8. The system of one of the preceding claims, **characterized in that** the information system (3) is an automated information system delivering patient information to the patient.

9. The system of one of the preceding claims, **characterized in that** the specified destination is a person familiar with the patient (P) or a specified communication terminal associated with a person familiar with the patient (P).

10. The system of one of the preceding claims, **characterized in that** at least one of the implantable medical device (1), the remote monitoring system (2) and the information system (3) is configured, for analyzing said at least one physiological parameter, to compare the at least one physiological parameter or a parameter value derived from the at least one physiological parameter to a threshold (T1, T2) and to generate said alert signal based on said comparison.

11. The system of one of the preceding claims, **characterized in that** the at least one physiological parameter includes a mean value of the heart rate or the heart rate at rest.

12. The system of claim 11, **characterized in that** said mean value is compared to a threshold (T1, T2) to generate said alert signal, wherein the threshold (T1, T2) is pre-defined or determined based on a group of prior values of the heart rate or the heart rate at rest.

13. The system of claim 12, **characterized in that** said threshold (T1, T2) is in a range between 90 bpm and 120 bpm.

14. The system of claim 12, **characterized in that** said threshold (T1, T2) is in a range between 110% and 150% of an average of the heart rate or the heart rate at rest over a predefined time period.

## Patentansprüche

1. System zum Erzeugen eines Alarms für eine systemische Infektion eines Patienten (P), wobei das System umfasst: eine implantierbare medizinische Vorrichtung (1), die so konfiguriert ist, dass sie mindestens einen physiologischen Parameter misst, ein Fernüberwachungssystem (2), das so konfiguriert ist, dass es Informationen von der implantierbaren medizinischen Vorrichtung (1) empfängt, und ein Informationssystem (3), das so konfiguriert ist, dass es mit dem Fernüberwachungssystem (2) kommuniziert, wobei mindestens eines von der implantierbaren medizinischen Vorrichtung (1), dem Fernüberwachungssystem (2) und dem Informationssystem (3) so konfiguriert ist, dass es Informationen analysiert, die sich auf den mindestens einen physiologischen Parameter beziehen, um ein Alarmsignal für eine systemische Infektion des Patienten (P) auf der Grundlage eines Zustands des mindestens einen physiologischen Parameters zu erzeugen, wobei mindestens eines von der implantierbaren medizinischen Vorrichtung (1), dem Fernüberwachungssystem (2) und dem Informationssystem (3) ferner so konfiguriert ist, dass es eine Alarmmeldung (B4, B5) erzeugt, die auf der Grundlage des Alarmsignals an ein spezifiziertes Ziel zu liefern ist, **dadurch gekennzeichnet, dass** das Informationssystem (3) so konfiguriert ist, dass es die Alarmmeldung unter Verwendung eines dedizierten Kommunikationspfads (A4, A5) an das Ziel überträgt, wobei das Informationssystem (3) so konfiguriert ist, dass es Informationen, die sich nicht auf die Alarmmeldung beziehen, auf einem Standard-Kommunikationspfad (A3) liefert, der sich von dem dedizierten Kommunikationspfad (A4, A5) unterscheidet.

2. Das System nach Anspruch 1, **dadurch gekennzeichnet, dass** die implantierbare medizinische Vorrichtung (1) eine Kommunikationsvorrichtung (17) umfasst zur Kommunikation mit dem Fernüberwachungssystem (2) unter Verwendung eines vordefinierten Kommunikationsprotokolls zum Übertragen von Informationen bezüglich des mindestens einen physiologischen Parameters an das Fernüberwachungssystem (2).

3. Das System nach Anspruch 2, **dadurch gekennzeichnet, dass** das Kommunikationsprotokoll MICS, BLE oder Zigbee ist.

4. Das System nach einem der Ansprüche 1 bis 3, **dadurch gekennzeichnet, dass** der mindestens eine physiologische Parameter mindestens eines aus folgendem umfasst: Körpertemperatur, Herzfrequenz, Herzfrequenz in Ruhe, Atemfrequenz, einen Körperaktivitätsparameter.

5. Das System nach einem der vorhergehenden Ansprüche, **dadurch gekennzeichnet, dass** die implantierbare medizinische Vorrichtung (1) so konfiguriert ist, dass es das Alarmsignal auf der Grundlage des mindestens einen physiologischen Parameters erzeugt und Informationen bezüglich des Alarmsignals an das Fernüberwachungssystem (2) überträgt.

6. Das System nach einem der vorhergehenden Ansprüche, **dadurch gekennzeichnet, dass** das Fernüberwachungssystem (2) so konfiguriert ist, dass es das Alarmsignal auf der Grundlage von Informationen bezüglich des mindestens einen physiologischen Parameters erzeugt und Informationen bezüglich des Alarmsignals an das Informationssystem (3) überträgt.

7. Das System nach einem der vorhergehenden Ansprüche, **dadurch gekennzeichnet, dass** das Informationssystem (3) so konfiguriert ist, dass es das Alarmsignal auf der Grundlage von Informationen erzeugt, die sich auf den mindestens einen physiologischen Parameter beziehen.

8. Das System nach einem der vorhergehenden Ansprüche, **dadurch gekennzeichnet, dass** das Informationssystem (3) ein automatisiertes Informationssystem ist, das dem Patienten Patienteninformationen liefert.

9. Das System nach einem der vorhergehenden Ansprüche, **dadurch gekennzeichnet, dass** das spezifizierte Ziel eine mit dem Patienten (P) vertraute Person oder ein spezifiziertes Kommunikationsendgerät, das mit einer mit dem Patienten (P) vertrauten Person verbunden ist.

10. Das System nach einem der vorhergehenden Ansprüche, **dadurch gekennzeichnet, dass** mindestens eines von der implantierbaren medizinischen Vorrichtung (1), dem Fernüberwachungssystem (2) und dem Informationssystem (3) zum Analysieren des mindestens einen physiologischen Parameters konfiguriert ist, um den mindestens einen physiologischen Parameter oder einen Parameterwert abgeleitet von dem mindestens einen physiologischen Parameter mit einem Schwellenwert (T1, T2) zu vergleichen und das Alarmsignal auf der Grundlage dieses Vergleichs zu erzeugen.

11. Das System nach einem der vorhergehenden Ansprüche, **dadurch gekennzeichnet, dass** der mindestens eine physiologische Parameter einen Mittelwert der Herzfrequenz oder der Herzfrequenz in Ruhe umfasst.

12. Das System nach Anspruch 11, **dadurch gekennzeichnet, dass** der Mittelwert mit einem Schwellenwert (T1, T2) verglichen wird, um das Alarmsignal zu erzeugen, wobei der Schwellenwert (T1, T2) vordefiniert ist oder auf der Grundlage einer Gruppe früherer Werte der Herzfrequenz oder der Herzfrequenz in Ruhe bestimmt wurde/ist.

13. Das System nach Anspruch 12, **dadurch gekennzeichnet, dass** der Schwellenwert (T1, T2) in einem Bereich zwischen 90 bpm und 120 bpm liegt.

14. Das System nach Anspruch 12, **dadurch gekennzeichnet, dass** der Schwellenwert (T1, T2) in einem Bereich zwischen 110% und 150% eines Durchschnitts der Herzfrequenz oder der Herzfrequenz in Ruhe über eine vordefinierte Zeitspanne liegt.

## Revendications

1. Système destiné à générer une alerte pour une infection systémique d'un patient (P), le système comprenant : un dispositif médical implantable (1) conçu pour mesurer au moins un paramètre physiologique, un système de surveillance à distance (2) conçu pour recevoir des informations du dispositif médical implantable (1), et un système d'information (3) conçu pour communiquer avec ledit système de surveillance à distance (2), dans lequel au moins l'un parmi le dispositif médical implantable (1), le système de surveillance à distance (2) et le système d'information (3) est conçu pour analyser les informations liées audit au moins un paramètre physiologique afin de générer un signal d'alerte pour une infection systémique du patient (P) en se basant sur un état d'au moins un paramètre physiologique, dans lequel au moins un parmi le dispositif médical implantable (1), le système de surveillance à distance (2) et le système d'information (3) est en outre conçu pour générer un message d'alerte (B4, B5) à pourvoir au niveau d'une destination spécifiée en se basant sur ledit signal d'alerte,**caractérisé en ce que** le système d'information (3) est conçu pour transmettre ledit message d'alerte à ladite destination en utilisant un trajet de communication dédié (A4, A5), dans lequel le système d'information (3) est conçu pour fournir des informations non liées audit message d'alerte sur un trajet de communication par défaut (A3) différent dudit trajet de communication dédié (A4, A5).

2. Système selon la revendication 1, **caractérisé en ce que** le dispositif médical implantable (1) comprend un dispositif de communication (17) pour communiquer avec le système de surveillance à distance (2) en utilisant un protocole de communication prédéfini destiné à transmettre des informations liées audit au moins un paramètre physiologique au système de surveillance à distance (2).

3. Système selon la revendication 2, **caractérisé en ce que** le protocole de communication est MICS, BLE ou Zigbee.

4. Système selon l'une des revendications 1 à 3, **caractérisé en ce qu'**au moins un paramètre physiologique inclut au moins un parmi la température corporelle, le rythme cardiaque, le rythme cardiaque au repos, la fréquence respiratoire, un paramètre d'activité corporelle.

5. Système selon l'une des revendications précédentes, **caractérisé en ce que** le dispositif médical implantable (1) est conçu pour générer ledit signal d'alerte en se basant sur au moins un paramètre physiologique et pour transmettre des informations liées audit signal d'alerte au système de surveillance à distance (2).

6. Système selon l'une des revendications précédentes, **caractérisé en ce que** le système de surveillance à distance (2) est conçu pour générer ledit signal d'alerte en se basant sur des informations liées à l'au moins un paramètre physiologique et pour transmettre des informations liées audit signal d'alerte au système d'information (3).

7. Système selon l'une des revendications précédentes, **caractérisé en ce que** le système d'information (3) est conçu pour générer ledit signal d'alerte en se basant sur des informations liées audit au moins un paramètre physiologique.

8. Système selon l'une des revendications précédentes, **caractérisé en ce que** le système d'information (3) est un système d'information automatisé distribuant les informations de patient au patient.

9. Système selon l'une des revendications précédentes, **caractérisé en ce que** la destination spécifiée est une personne familière du patient (P) ou un terminal de communication spécifié associé à une personne familière du patient (P).

10. Système selon l'une des revendications précédentes, **caractérisé en ce qu'**au moins un parmi le dispositif médical implantable (1), le système de surveillance à distance (2) et le système d'information (3) est conçu pour analyser ledit au moins un paramètre physiologique, afin de comparer l'au moins un paramètre physiologique ou une valeur de paramètre dérivée de l'au moins un paramètre physiologique à un seuil (T1, T2) et de générer ledit signal d'alerte en se basant sur ladite comparaison.

11. Système selon l'une des revendications précédentes, **caractérisé en ce que** l'au moins un paramètre physiologique inclut une valeur moyenne du rythme cardiaque ou du rythme cardiaque au repos.

12. Système selon la revendication 11, **caractérisé en ce que** ladite valeur moyenne est comparée à un seuil (T1, T2) pour générer ledit signal d'alerte, dans lequel le seuil (T1, T2) est prédéfini ou déterminé en se basant sur un groupe de valeurs antérieures du rythme cardiaque ou du rythme cardiaque au repos.

13. Système selon la revendication 12, **caractérisé en ce que** ledit seuil (T1, T2) est dans une plage entre 90 bpm et 120 bpm.

14. Système selon la revendication 12, **caractérisé en ce que** ledit seuil (T1, T2) est dans une plage entre 110 % et 150 % d'une moyenne du rythme cardiaque ou du rythme cardiaque au repos sur une période prédéfinie.
